(19) Europäisches Patentamt
European Patent Office
Office européen des brevets

(11) Publication number: 0 330 117 A2

# (12) EUROPEAN PATENT APPLICATION

(21) Application number: 89102896.1

(22) Date of filing: 20.02.89

(51) Int. Cl.4: C08F 283/10

(30) Priority: 22.02.88 JP 39185/88

(43) Date of publication of application: 30.08.89 Bulletin 89/35

(84) Designated Contracting States: DE FR GB

(71) Applicant: MITSUBISHI RAYON CO. LTD.
3-19, Kyobashi 2-chome Chuo-Ku
Tokyo 104(JP)

(72) Inventor: Mukai, Nobuhiro
13-19-105, Inokuchidai 1-chome, Nishi-ku
Hiroshima-shi, Hiroshima-ken(JP)
Inventor: Ige, Hitoshi
2-6-206, Kurokawa 3-chome, Ohtake-shi
Hiroshima-ken(JP)

(74) Representative: Patentanwälte TER MEER - MÜLLER - STEINMEISTER
Mauerkircherstrasse 45
D-8000 München 80(DE)

## (54) Photopolymerizable composition.

(57) A photopolymerizable composition here disclosed is useful as a material for dental prostheses and denture-bases, and the composition comprises an alicyclic epoxy compound as the main component, methyl methacrylate as a diluent, a cationic photopolymerization initiator, and a photosensitizer selected from perylene or naphthacene. A photocured material of the above-mentioned composition is excellent in glossiness, wear resistance and plaque resistance.

EP 0 330 117 A2

# Photopolymerizable Composition

## BACKGROUND OF THE INVENTION

### (1) Field of the Invention

The present invention relates to a photopolymerizable composition, particularly to a composition which can be used for a photo-setting treatment on the surfaces of plastic dental prostheses and denture-bases. More specifically, the present invention relates to a composition which permits remarkably improving smoothness, glossiness and wear resistance of the surfaces of plastic dental prostheses and denture-bases, when photo-cured, and which also permits providing the surfaces of the plastic dental prostheses and denture-bases with the ability to prevent the adhesion of plaque which results from bacteria contamination.

### (2) Description of the Prior Art

Plastic dental prostheses and denture-bases can be more easily manufactured than ceramic dental prostheses and the like, and they are relatively inexpensive. For these reasons, the plastic dental prostheses and denture-bases are widely utilized. However, for example, the dental prostheses made from polymethyl methacrylate, polycarbonate and the like have poorer wear resistance than the ceramic dental prostheses, and thus if used for a long period of time, they are easily worn down by the friction of a toothbrush or the like, and the gloss on the surfaces thereof falls off and the beautiful appearance thereof is impaired. In addition, it is also indicated as a problem that the dental prostheses become uncomfortable to wear.

As a method for eliminating such drawbacks, the same applicant as in the present application has already suggested a photo-radical polymerizable composition which comprises a blend of a multifunctional (meth)acrylate such as dipentaerythritol tetraacrylate or trimethylolpropane acrylate and a photopolymerization initiator such as a benzoin alkyl ether (Japanese Patent Publication Nos. 21660/1986 and 58180/1986).

A cured coating film of this suggested composition is excellent in wear resistance, but when the cured coating film is used in a mouth for a long period of time, plaque adheres thereto as a result of bacteria contamination, so that smoothness and glossiness on the surface of the coating film deteriorate noticeably, the beautiful appearance thereof is impaired, and sanitary problems occur.

## SUMMARY OF THE INVENTION

A first object of the present invention is to provide dental prostheses or denture-bases having excellent wear resistance, maintaining surface gloss and involving less wear uncomfortability.

Another object of the present invention is to provide dental prostheses or denture-bases onto which less plaque adheres and on which smoothness and glossiness are maintained, even when they are used for a long period of time.

The objects of the present invention can be achieved by providing a photopolymerizable composition in which 100 parts by weight of an alicyclic epoxy compound is blended with 0.1 to 10 parts by weight of a cationic photopolymerization initiator, 1 to 99 parts by weight of methyl methacrylate and 0.001 to 10 parts by weight of at least one selected from perylene and naphthacene as a photosensitizer.

## DETAILED DESCRIPTION OF THE INVENTION

A preferable alicyclic epoxy compound used in the present invention include compounds having an epoxycycloalkyl group, and the carbon number of the alkyl group in the epoxy compound usually used is in the range of 5 to 8. Above all, the compounds having epoxycyclohexyl group are particularly preferable.

Typical examples of the preferably usable alicyclic epoxy compound include bis(3,4-epoxycyclohexyl)

adipate and 3,4-epoxycyclohexylmethyl-3',4'-epoxycyclohexane carboxylate represented by the following structural formulae, and bis(3,4-epoxycyclohexyl) adipate is more preferable:

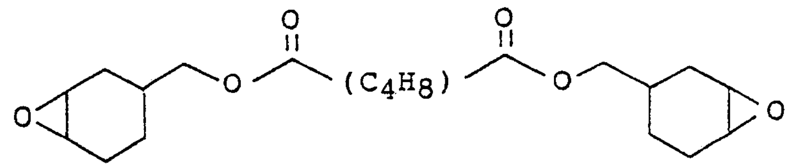

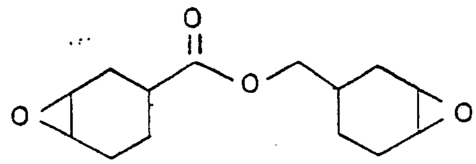

As a cationic photopolymerization initiator used in the present invention, an aromatic sulfonium salt represented by the following general formula (I) is preferable:

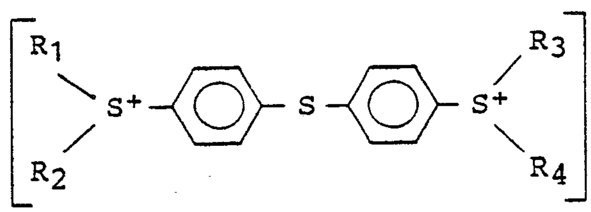 2MX$^-$ (I)

wherein each of $R_1$ to $R_4$ is a hydrogen atom, an alkyl group having 1 to 10 carbon atoms, a phenyl group or a hydroxyalkyl group having 1 to 10 carbon atoms, and $R_1$ to $R_4$ may be identical or different; and MX is at least one selected from the group consisting of $SbF_6$, $AsF_6$, $PF_6$ and $BF_4$.

Typical examples of the aromatic sulfonium salt include a compound in which $R_1$ is a phenyl group, $R_2$ is a hydrogen atom, each of $R_3$ and $R_4$ is a 2-hydroxyethyl group, and the counter anion MX is $SbF_6$, i.e., [4-(di-β-hydroxyethylsufonyo)phenyl-4'-(phenylsulfonyo)phenyl] sulfide•bishexafluoro antimonate; a compound in which the counter anion MX in the above-mentioned compound is $AsF_6$, $PF_6$ or $BF_4$; a compound in which $R_1$ is a phenyl group, $R_2$ is a hydrogen atom, $R_3$ is a methyl group, $R_4$ is a 3-hydroxypropyl group, and the counter anion MX is $SbF_6$; a compound in which $R_1$ is a phenyl group, $R_2$ is a hydrogen atom, $R_3$ is a propyl group, $R_4$ is a 2-hydroxyethyl group, and the counter anion MX is $SbF_6$; a compound in which in the above-mentioned compound, $R_3$ is a propyl group and $R_4$ is a 2-hydroxyethyl group; a compound in which in the above-mentioned compound, $R_3$ is a propyl group and $R_4$ is a methyl group; a compound in which both of $R_1$ and $R_2$ are phenyl groups or hydrogen atoms, $R_3$ is a propyl group, and $R_4$ is a 2-hydroxyethyl group; a compound in which $R_1$ is a phenyl group, $R_2$ is a propyl group, $R_3$ is a hydrogen atom, $R_4$ is a 2-hydroxyethyl group; and a compound in which $R_1$ is a phenyl group, $R_2$ is a 2-hydroxyethyl group, $R_3$ is a hydrogen atom, $R_4$ is a propyl group. Of these compounds, the first mentioned compound is preferable, because when the composition of the present invention in which this compound is used is photopolymerized, excellent physical properties can be obtained. The cationic photopolymerization initiator, when used, can be dissloved in a reactive diluent to heighten stability, and one example of this reactive diluent is propylene carbonate.

In the present invention, the addition of a photosensitizer is necessary with the intention of providing the composition with high curability. Examples of the usable photosensitizer include perylene and naphthacene, and both of them may be used together. Of the two photosensitizers, perylene is more preferable in point of functional effect.

The amount of the cationic photopolymerization initiator is in the range of 0.1 to 10 parts by weight, preferably 0.2 to 8 parts by weight, more preferably 0.4 to 6 parts by weight with respect to 100 parts by weight of the alicyclic epoxy compound.

In the present invention, for the purposes of lowering the viscosity of the system and providing the composition with coating properties, methyl methacrylate is blended as a diluent in an amount of 1 to 99 parts by weight, preferably 20 to 80 parts by weight with respect to 100 parts by weight of the above-mentioned alicyclic epoxy compound. In this manner, when the alicyclic epoxy compound is combined with

methyl methacrylate, good plaque resistance can be obtained.

Furthermore, in the present invention, at least one selected from perylene and naphthacene is used as the photosensitizer in an amount of 0.001 to 10 parts by weight, preferably 0.05 to 2 parts by weight with respect to 100 parts by weight of the alicyclic epoxy compound. It has been appreciated that perylene and naphthacene have no carcinogenicity, and thus they are acceptable as the suitable photosensitizer in the field of dental materials. Surprisingly, the photosensitization effect of perylene and naphthacene permits obtaining high curability and long-term plaque resistance, and so, this composition containing the photosensitizer is suitable for use in a mouth.

In curing the composition of the present invention, wave length in the range of ultraviolet to visible light can be used. As the typical light source for emitting such wave length, there can be used a halogen lamp, xenon lamp, mercury lamp or fluorescent lamp.

Additionally, in the present invention, an inorganic material such as colloidal silica, a colorant, and the like can be used as the needs of the case demands.

When the composition of the present invention just described in detail is photocured, the photocured material can be obtained which is excellent in glossiness and wear resistance. Furthermore, when dental prostheses, denture-bases and the like are coated with the composition of the present invention and the composition is then photocured, the photocured material which is remarkably excellent in plaque resistance can be obtained. Therefore, it is fair to say that the composition of the present invention is particularly effective and useful in the field of dental materials.

Now, the present invention will be further described in detail in accordance with examples, but the scope of the present invention should not be limited to these examples.

## Example 1

A 2-mm-thick, 20-mm-long, 20-mm-wide polymethyl methacrylate substrate was polished with a water-resistant polishing paper of No. 320 (JIS standard), washed by the utilization of ultrasonic wave, dried, and washed with ethyl alcohol. On the other hand, a photopolymerizable composition was prepared by blending 100 parts by weight of bis(3,4-epoxycyclohexyl) adipate (trade name ERL-4299; made by Union Carbide Corp.) as an alicyclic epoxy compound with 2.5 parts by weight of [4-(di-β-hydroxyethylsulfonyo)phenyl-4′-(phenylsulfonyo)phenyl] sulfide•bishexafluoro antimonate) (trade name UVI-6970; made by Union Carbide Corp.) as a cationic photopolymerization initiator, 50 parts by weight of methyl methacrylate as a diluent and 0.05 part by weight of perylene as a photosensitizer. The above-mentioned polymethyl methacrylate substrate was then coated with the thus prepared composition by the use of a brush, and it was then allowed to stand for several minutes. Afterward, the composition thereon was irradiated with visible light by the use of a visible light irradiator (trade name Labolight LV-I; made by GC Dental Industry Co., Ltd.) for 15 minutes in order to polymerize/cure the composition.

For the thus prepared specimen, a toothbrush wear resistance test (load 500 g, 3,000 stroke) was carried out in a 50% tooth-polishing agent slurry. Surface glossiness was evaluated by a glossimeter and surface state was observed by an optical microscope. The results are set forth in Table 1.

## Example 2

Following the same procedure as in Example 1 with the exception that the trade name ERL-4299 of an alicyclic epoxy compound was replaced with 3,4-epoxycyclohexylmethyl-3′,4′-epoxycyclohexane carboxylate (trade name ERL-4221; made by Union Carbide Corp.), a composition was prepared. After polymerization/curing of the composition, evaluation was made, and the results are set forth in Table 1.

## Examples 3 and 4

Each composition was prepared following the same procedure as in Example 1 with the exception that an aromatic sulfonium salt represented by the following formula was used as a cationic photopolymerization initiator:

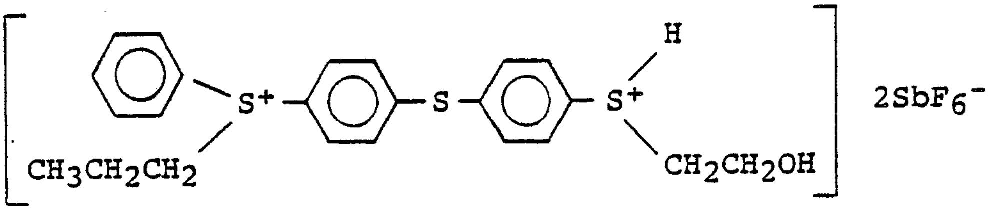

or

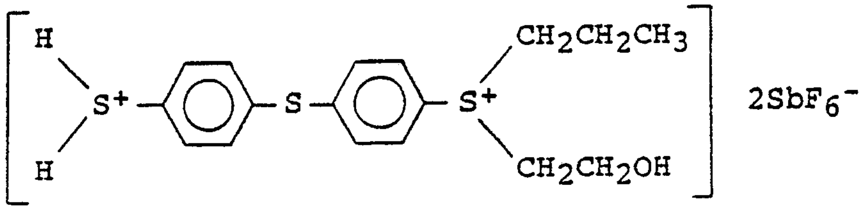

Afterward, polymerization/curing and evaluation were performed in the same manner as in Example 1.

Examples 5 and 6

Following the same procedure as in Example 1 with the exception that the amount of the cationic photopolymerization initiator of the trade name UVI-6970 was changed to 0.5 and 5.0 parts by weight, compositions were prepared. Afterward, polymerization/curing and evaluation were performed in the same manner as in Example 1.

Examples 7 to 10

Following the same procedure as in Example 1 with the exception that the amount of methyl methacrylate was changed to 5, 95, 25 and 75 parts by weight, compositions were prepared. Afterward, polymerization/curing and evaluation were performed in the same manner as in Example 1.

Example 11

Following the same procedure as in Example 1 with the exception that as a photosensitizer, perylene was replaced with naphthacene, a composition was prepared. Afterward, polymerization/curing and evaluation were performed in the same manner as in Example 1.

Examples 12 and 13

Following the same procedure as in Example 1 with the exception that the amount of the photosensitizer perylene was changed to 0.01 and 1.0 part by weight, compositions were prepared. Afterward, polymerization/curing and evaluation were performed in the same manner as in Example 1.

Example 14

Following the same procedure as in Example 1 with the exception that 10 parts by weight of colloidal silica (trade name R-972; made by Degussa AG) was further blended, a composition was prepared. Afterward, polymerization/curing and evaluation were performed in the same manner as in Example 1.

The results of Examples 2 to 14 are set forth all together in Table 1.

Comparative Example 1

For the substrate only polished with a water-resistant polishing paper of No. 320 which was used in Example 1 but which was not coated with the composition of the present invention, evaluation was made as in Example 1. The results are set forth in Table 1.

Comparative Example 2

A composition was prepared following the same procedure as in Example 1 with the exception that the trade name ERL-4299 of an epoxy compound was replaced with four-functional polyglycidylamine represented by the formula:

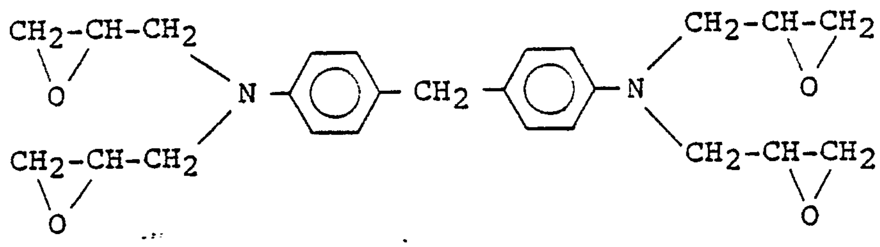

Afterward, polymerization/curing and evaluation were performed in the manner as in Example 1.

Comparative Example 3

Following the same procedure as in Example 1 with the exception that perylene of a photosensitizer was not blended, a composition was prepared. Afterward, polymerization/curing and evaluation were performed in the same manner as in Example 1.

Comparative Examples 4 and 5

Following the same procedure as in Example 1 with the exception that methyl methacrylate was not blended and that 200 parts by weight of methyl methacrylate was blended, compositions were prepared. Afterward, polymerization/curing and evaluation were performed in the same manner as in Example 1.

The results of Comparative Examples 2 to 5 are set forth all together in Table 1.

Table 1

| | Gloss Degree (relative value) | Wear Resistance*1 | Remarks |
|---|---|---|---|
| Example 1 | 90 | O | |
| Example 2 | 91 | O | |
| Example 3 | 91 | O | |
| Example 4 | 90 | O | |
| Example 5 | 90 | O | |
| Example 6 | 90 | O | |
| Example 7 | 91 | O | |
| Example 8 | 90 | O | |
| Example 9 | 91 | O | |
| Example 10 | 90 | O | |
| Example 11 | 91 | O | |
| Example 12 | 91 | O | |
| Example 13 | 90 | O | |
| Example 14 | 90 | O | |
| Comp. Ex. 1 | 65 | X | |
| Comp. Ex. 2 | - | - | uncured |
| Comp. Ex. 3 | 88 | Δ | |
| Comp. Ex. 4 | 87 | Δ | coating layer was uneven |
| Comp. Ex. 5 | 89 | Δ | coating layer was uneven |

*1 According to the observation through a microscope, ranking was made as follows:
O: Even shallow grooves were scarcely observed.
Δ: Shallow grooves were slightly observed.
X: Many deep grooves were observed.

Examples 15 to 28

The surface of a polymethyl methacrylate denture-base was washed with ethyl alcohol, and the cleaned

denture-base was then coated with the photopolymerizable composition used in Examples 1 to 14. Afterward, the compositions thereon were polymerized/cured by light irradiation in the same manner as in Example 1.

Afterward, each denture-base was actually set to the upper portion in a mouth, and the situation of plaque adhesion was observed after one week and after 3 months by the use of a plaque dyeing liquid (trade name Prospec; made by GC Dental Industry Co., Ltd.). The results are set forth in Table 2. It is apparent from the results that the surface of each denture-base was not dyed at all even after 3 months, which meant that the cured coating film of the composition of the present case had remarkably excellent plaque resistance.

Comparative Examples 6 and 7

The same procedure as in Example 15 was repeated with the exception that photopolymerizable compositions used in Comparative Examples 3 and 4 were used, and a plaque resistance test was then carried out in the same manner as in Example 15. The results are set forth in Table 2.

Comparative Examples 8 and 9

The same procedure as in Example 15 was repeated with the exception that methyl methacrylate was replaced with chloroform and styrene, in order to obtain photopolymerizable compositions. For these compositions, a plaque resistance test was carried out in the same manner as in Example 15. The results are set forth in Table 2.

Table 2

| | Used Composition | Plaque Resistance*2 After 1 Week | After 3 Months |
|---|---|---|---|
| Example 15 | composition in Example 1 | O | O |
| Example 16 | composition in Example 2 | O | O |
| Example 17 | composition in Example 3 | O | O |
| Example 18 | composition in Example 4 | O | O |
| Example 19 | composition in Example 5 | O | O |
| Example 20 | composition in Example 6 | O | O |
| Example 21 | composition in Example 7 | O | O |
| Example 22 | composition in Example 8 | O | O |
| Example 23 | composition in Example 9 | O | O |
| Example 24 | composition in Example 10 | O | O |
| Example 25 | composition in Example 11 | O | O |
| Example 26 | composition in Example 12 | O | O |
| Example 27 | composition in Example 13 | O | O |
| Example 28 | composition in Example 14 | O | O |
| Comp. Ex. 6 | composition in Comp. Ex. 3 | O | X |
| Comp. Ex. 7 | composition in Comp. Ex. 4 | O | X |
| Comp. Ex. 8 | - | O | X |
| Comp. Ex. 9 | - | O | X |

*2 According to the observation of the adhesion situation of the plaque dyeing liquid, ranking was made as follows:
- O: The denture-base was not dyed at all, which means that the plaque resistance was good.
- X: The densture-base was dyed, which means that the plaque resistance was bad.

## Claims

1. A photopolymerizable composition in which 100 parts by weight of an alicyclic epoxy compound is blended with 0.1 to 10 parts by weight of a cationic photopolymerization initiator, 1 to 99 parts by weight of methyl methacrylate and 0.001 to 10 parts by weight of at least one selected from perylene and naphthacene as a photosensitizer.

2. A photopolymerizable composition according to Claim 1 wherein said alicyclic epoxy compound is bis(3,4-epoxycyclohexyl) adipate or 3,4-epoxycyclohexylmethyl-3′,4′-epoxycyclohexane carboxylate.

3. A photopolymerizable composition according to Claim 1 wherein said cationic photopolymerization initiator is an aromatic sulfonium salt represented by the general formula (I)

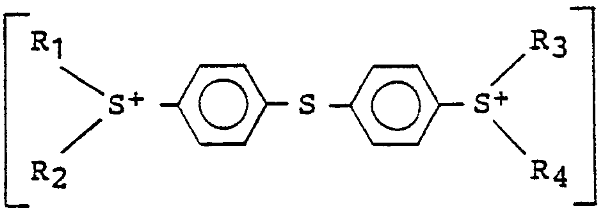 $2MX^-$ (I)

wherein each of $R_1$ to $R_4$ is a hydrogen atom, an alkyl group having 1 to 10 carbon atoms, a phenyl group or a hydroxyalkyl group having 1 to 10 carbon atoms, and $R_1$ to $R_4$ may be identical or different; and MX is at least one selected from the group consisting of $SbF_6$, $AsF_6$, $PF_6$ and $BF_4$.

4. A photopolymerizable composition according to Claim 1 wherein said cationic photopolymerization initiator is [4-(di-β-hydroxyethylsulfonio)phenyl-4′-(phenylsulfonyo)-phenyl] sulfide•bishexafluoro antimonate.

5. A photopolymerizable composition according to Claim 1 wherein said cationic photopolymerization initiator is an aromatic sulfonium salt represented by the formula (2) or (3):

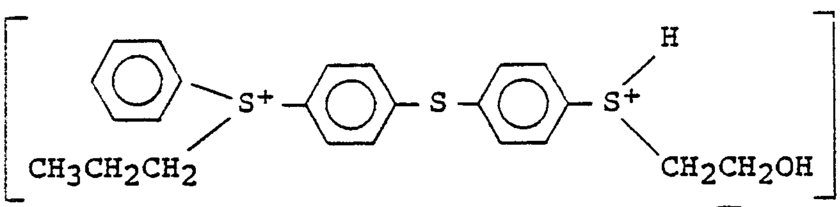 $2SbF_6^-$ (2)

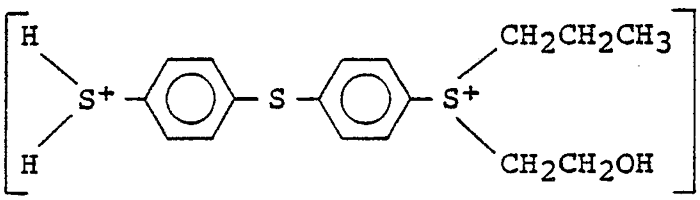 $2SbF_6^-$ (3)